(19) Europäisches Patentamt
European Patent Office
Office européen des brevets

(11) **EP 2 962 741 B1**

(12) **EUROPEAN PATENT SPECIFICATION**

(45) Date of publication and mention of the grant of the patent:
**17.05.2017 Bulletin 2017/20**

(21) Application number: **14757463.6**

(22) Date of filing: **21.02.2014**

(51) Int Cl.:
*G06K 9/00* (2006.01)      *A61B 5/00* (2006.01)
*A61B 5/11* (2006.01)

(86) International application number:
**PCT/JP2014/054140**

(87) International publication number:
**WO 2014/132885 (04.09.2014 Gazette 2014/36)**

(54) **GOLF IMPLEMENT FITTING SYSTEM AND GOLF IMPLEMENT FITTING PROGRAM**

PASSSYSTEM FÜR GOLFVORRICHTUNG UND PASSPROGRAMM FÜR GOLFVORRICHTUNG

SYSTÈME D'INSTALLATION D'INSTRUMENT DE GOLF ET PROGRAMME D'INSTALLATION D'INSTRUMENT DE GOLF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2013   JP 2013037493**
**10.04.2013   JP 2013082102**

(43) Date of publication of application:
**06.01.2016   Bulletin 2016/01**

(73) Proprietor: **Mitsubishi Rayon Co., Ltd.**
**Tokyo 100-8253 (JP)**

(72) Inventors:
• **KODAMA, Hitoshi**
**Kashiwa-shi**
**Chiba 277-8568 (JP)**
• **SUZUKI, Katsuyuki**
**Kashiwa-shi**
**Chiba 277-8568 (JP)**
• **SHIMONO, Satoshi**
**Toyohashi-shi**
**Aichi 440-8601 (JP)**

(74) Representative: **Hoffmann Eitle**
**Patent- und Rechtsanwälte PartmbB**
**Arabellastraße 30**
**81925 München (DE)**

(56) References cited:
WO-A1-2006/100712      JP-A- H06 210 027
JP-A- 2008 171 280      JP-A- 2010 131 442
JP-A- 2010 240 209      JP-A- 2011 000 425
JP-A- 2011 086 199      US-A1- 2004 127 303
US-A1- 2006 211 510

• SHIN ISOBE ET AL.: 'Optimal Design of Golf Shaft Using Response Surface Method' PROCEEDINGS OF THE CONFERENCE ON COMPUTATIONAL ENGINEERING AND SCIENCE vol. 13, no. 1, 19 May 2008, pages 473 - 474, XP008177766

EP 2 962 741 B1

**Description**

Field of the Invention

The present invention relates to a golf equipment fitting system and a golf equipment fitting program.
Priority is claimed on Japanese Patent Application No. 2013-37493, filed February 27, 2013, and Japanese Patent Application No. 2013-82102, filed April 10, 2013.

Background Art

In 2008, restitution regulations on golf club heads were enacted by the Rules of Golf, making it difficult to optimize distance by the head alone. In response to the regulations, manufacturers have switched their focus to the shaft and have attempted to improve carry by making the most of the bending of a shaft. Thus, as of 2012, varieties of shafts have increased, resulting in a further increase in varieties of clubs derived from combinations of heads and shafts. Accordingly, when a player purchases a club, it is difficult for the player to select the most suitable equipment, especially the most suitable shaft for himself.
Fitting technologies are intended to solve the above problem. Patent Publication 1 discloses an example of a fitting technology that focuses on the entire golf club. The behavior of a head at the moment of impact is photographed by a high-speed camera, and the data are converted to three-dimensional coordinates by a DLT method so that the position of the head is quantified. By so doing, the head position at the time of impact is specified for each club, enabling a golfer to choose a proper club. However, it is necessary for the golfer to use a large number of clubs when actually hitting golf balls. Thus, even though a selection of preferable clubs is made available, it is still difficult for a golfer to choose a club that is best suited to himself.
The technology described in Patent Publication 2 is meant to solve the above problem. First, a golfer's swing is measured, and the head behavior of a golf club is simulated based on the swing data. By performing only one swing measurement, the golfer can obtain a result that corresponds to hundreds of test hits. However, when a golfer uses clubs with different stiffnesses and weights, the golfer hits a ball by changing the swing itself depending on the club. Thus, when the head behavior is simulated on clubs or shafts with significantly different properties based on the data obtained by one swing, the simulation results may not provide actual solutions.
The technology described in Patent Publication 3 is meant to solve the above problem. Using response surface methodology, the technology calculates swings which are different depending on shaft properties (three properties: flex, kick point, torque), and performs simulations based on the swing data modified accordingly. As a result, the technology provides simulations that are even closer to actual conditions.

PRIOR ART PUBLICATION

PATENT PUBLICATION

Patent Publication 1: JP2005-312734A
Patent Publication 2: JP4871218B
Patent Publication 3: JP2011-425A

SUMMARY OF THE INVENTION

PROBLEMS TO BE SOLVED BY THE INVENTION

Using the technology described in Patent Publication 3, it is difficult to simulate by changing the "weights" and "lengths" of golf club components, for example. That is because when the weight or length is changed, even for the same golfer, swing time differs significantly from the position at address (when a golf club is in contact with the ground) to the top (the moment the golf club is in full backswing), and from the position at the top to impact (the moment the head touches the ball), and so forth, and sufficiently accurate calculation results cannot be obtained. Thus, the technology described in Patent Publication 3 is limited to being applied to golf club specifications excluding those that significantly affect swing time such as the weight or length of a club.
In addition, it is difficult to instantly find appropriate specifications from massive calculation results when the technology in Patent Publication 3 is used. Thus, it is difficult to apply the technology to a fitting system that requires instant feedback.

The present invention was carried out in consideration of the problems above. The objective is to provide a golf equipment fitting system and a golf equipment fitting program capable of providing accurate calculation results even when specifications that affect swing time are changed, and of outputting simulation results in a short period of time.

SOLUTIONS TO THE PROBLEMS

In the following, numerical references as shown in the accompanying drawings are provided in parentheses for an easier understanding of the present invention. However, the present invention is not limited to the embodiments shown in the drawings.

To solve the aforementioned problems, an aspect of the present invention is a golf equipment fitting system (2) provided with the following: a swing data acquisition unit (21) that acquires swing data from a sensor attached to multiple golf clubs with different specifications; a swing response surface computation unit (221) that calculates a swing response surface using the response surface methodology based on the swing data obtained by the swing data acquisition unit; a time response surface computation unit (222) that calculates a time response surface using the response surface methodology based on the swing data obtained by the swing data acquisition unit; a simulation unit (23) that calculates the swing data of a golf club that is not used for measurement from the swing response surface and the time response surface and simulates the swing of the golf club based on the obtained swing data; and a results output unit (24) that outputs the results of simulations conducted by the simulation unit.

Another aspect of the present invention is characterized in that, in the golf equipment fitting system, the specifications of multiple golf clubs to be used in simulations include at least the weight of a shaft.

Yet another aspect of the present invention is characterized in that, in the golf equipment fitting system, the specifications of multiple golf clubs to be used in simulations include at least the length of a club.

Yet another aspect of the present invention is characterized in that, in the golf equipment fitting system, the specifications of multiple golf clubs to be used in simulations include at least two different specifications, and the difference in impact degrees of the two different specifications is within a predetermined value.

Yet another aspect of the present invention is characterized in that, in the golf equipment fitting system, the specifications of multiple golf clubs to be used in simulations are nine specifications selected from among 27 specifications obtained in combination of three different specifications and three levels each of the three specifications based on an L9 orthogonal array for the design of experiments.

Yet another aspect of the present invention is characterized in that, in the golf equipment fitting system, the three different specifications are weight, kick point and flex.

Yet another aspect of the present invention is characterized in that, in the golf equipment fitting system, the three different specifications are selected from among the stiffness, length, total weight and balance of a club.

Yet another aspect of the present invention is characterized in that, in the golf equipment fitting system, the three different specifications are selected from among the height, depth, distance and angle of the center of gravity of the head.

Yet another aspect of the present invention is characterized in that, in the golf equipment fitting system, the three different specifications are the loft angle, lie angle and face angle of a club head.

Yet another aspect of the present invention is characterized in that, in the golf equipment fitting system, when the three different specifications are divided into their respective levels, and when the number of levels of a first specification is set as (i) and one level as (1), the number of levels of a second specification is set as (j) and one level as (m), and the number of levels of a third specification is set as (k) and one level as (n), the results output unit allocates a result number defined by (l-1)i+(m-1)j+(n-1)k to each of the simulation results, then, each result number is replaced with a plotted number, which is the value obtained by subtracting (j-1)×k×(l-1) from the result number, and finally the results output unit outputs data as the fitting results by entering the plotted numbers on the horizontal axis and the simulation results on the vertical axis.

Yet another aspect of the present invention is characterized in that, in the golf equipment fitting system, the results output unit outputs the fitting results as line segments by connecting the plotted data applicable to the following conditions (i)~(iii):

(i) data in which a first specification is different from second and third specifications while second and third specifications are the same;
(ii) data in which a second specification is different from first and third specifications while first and third specifications are the same; and
(iii) data in which a third specification is different from first and second specifications while first and second specifications are the same.

Yet another aspect of the present invention is characterized in that, in the golf equipment fitting system, the results output unit outputs fitting results by converting the results to a natural language in association with corresponding absolute values and inclinations.

Yet another aspect of the present invention is characterized in that, in the golf equipment fitting system, the results output unit outputs a specification that exhibits the maximum value in simulation results by converting the specification to a product name.

Yet another aspect of the present invention is characterized in that, in the golf equipment fitting system, the results output unit selects and displays a golf club based on the results of simulations conducted by the simulation unit.

To solve the aforementioned problems, yet another aspect of the present invention is a golf equipment fitting program including the following steps that are implemented by a computer: a swing data acquisition step to acquire swing data by attaching a sensor to multiple golf clubs with different specifications; a swing response surface computation step to calculate a swing response surface using the response surface methodology based on the swing data obtained by the swing data acquisition step; a time response surface computation step to calculate a time response surface using the response surface methodology based on the swing data obtained by the swing data acquisition step; a simulation step to calculate the swing data of a golf club that is not used for measurement from the swing response surface and the time response surface and to simulate the swing of the golf club based on the obtained swing; and a results outputting step to output the results of simulations conducted by the simulation step.

EFFECTS OF THE INVENTION

According to the present invention, even when specifications that may affect swing time are changed, accurate computation results are obtained and simulation results are outputted in a short period of time.

BRIEF DESCRIPTION OF THE DRAWINGS

Fig. 1 shows a diagram showing the structure of a first embodiment of the present invention;
Fig. 2 shows views illustrating examples of specifications of a golf shaft;
Fig. 3 shows views illustrating examples of specifications of a golf shaft and club heads;
Fig. 4 is a table showing specifications of nine golf clubs for test hitting to obtain swing data;
Fig. 5 shows a flowchart showing operations of the golf equipment fitting system 2 shown in Fig. 1;
Fig. 6 is a graph showing swing data when shaft weights are the same;
Fig. 7 is a graph showing swing data when shaft weights are different;
Fig. 8 is a graph showing swing data prepared using only a swing response surface;
Fig. 9 is a graph showing swing data prepared using both a swing response surface and a time response surface;
Fig. 10 is a graph showing simulation results without applying a specific process;
Fig. 11 is a table showing relationships of simulation numbers and specifications of golf clubs;
Fig. 12 is a table showing relationships of result numbers and specifications of golf clubs after result numbers are assigned;
Fig. 13 is a graph showing simulation results obtained by entering result numbers on the horizontal axis;
Fig. 14 is a graph showing simulation results obtained by entering plotted numbers on the horizontal axis;
Fig. 15 is a table schematically showing nine different trajectories;
Fig. 16 is a graph showing simulation results obtained by entering the F values as complex conditions on the vertical axis;
Fig. 17 is a table showing impact degrees of specifications; and
Fig. 18 is a graph showing the results of simulations conducted by selecting the head weight, torsional rigidity distribution of a shaft and height of the center of gravity of a head as three different specifications.

DETAILED DESCRIPTION OF THE EMBODIMENTS

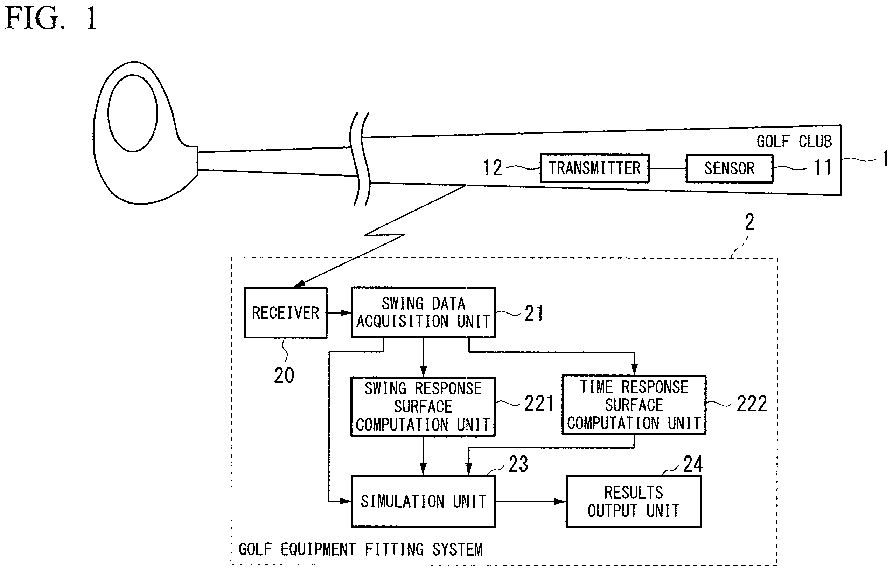

In the following, a golf equipment fitting system is described according to an embodiment of the present invention by referring to the accompanying drawings. Here, golf shaft specifications are listed as specification examples of golf equipment, but they are not the only options. Fig. 1 is a diagram showing the structure of the present embodiment. In the drawing, numerical reference 1 indicates a golf club where a golf shaft with known specifications is attached thereon, and is provided with sensor 11 inserted in the grip section of the shaft, and transmitter 12 that transmits the output signal from sensor 11 to the outside through wireless communication. Sensor 11 may be installed outside the shaft. Sensor 11 is a 6-axis sensor that detects and outputs 3-axis acceleration and 3-axis angular velocity. Sensor 11 may also be a 9-axis sensor to measure 3-axis orientations using geomagnetism in addition to 3-axis acceleration and 3-axis angular velocity. Alternatively, a 6-axis sensor and 3-axis geomagnetism sensor may be used together as sensor 11.

Numerical reference 2 is a golf equipment fitting system using a computer. Numerical reference 20 is a receiver unit that receives data outputted by sensor 11 (swing data) transmitted by transmitter 12. Numerical reference 21 is a swing data acquisition unit that acquires swing data through receiver unit 20. Numerical reference 221 is a swing response surface computation unit which calculates a swing response surface based on the swing data when a golf club 1 is test hit. Numerical reference 222 is a time response surface computation unit which calculates a swing time response surface based on the swing data when a golf club 1 is test hit. Numerical reference 23 is a simulation unit which conducts simulations by FEM (Finite Element Method) using the swing response surface and time response surface. Numerical reference 24 is a results output unit which outputs the results of simulations conducted in simulation unit 23.

Next, a golf club 1 is described. Golf club 1 is used for test hitting to obtain swing measurement data. When a golfer swings a club, the golfer adjusts the swing according to club properties such as the shaft weight, flexural rigidity and torsional rigidity. Thus, if swing measurement data are obtained for analysis by using a golf club with specific properties, it is difficult to achieve proper analysis results. That is because swings differ depending on club specifications. Thus, based on the design of experiments, three different specifications are selected from numerous club specifications, and 27 golf clubs are prepared by applying three levels each to the three specifications. Among the 27 golf clubs, nine clubs are selected based on the L9 orthogonal array. To obtain 3-factor and 3-level data without using the design of experiments, it is necessary to conduct experiments on $3^3$=27 golf clubs, and such procedures are not practical. Here, as examples of specifications, the following are picked: the weight of a shaft, the flexural rigidity of a shaft (among shaft specifications corresponds to the flex, hereinafter referred to as "flex"), and the flexural rigidity distribution of the shaft (among shaft specifications corresponds to kick points, hereinafter referred to as "kick point"). Three specifications of a golf club to be used here each have three levels. However, they are not the only options, and any other clubs may be used as long as they have multiple different specifications. The present embodiment is preferably applied to golf clubs with specifications of different weights and lengths.

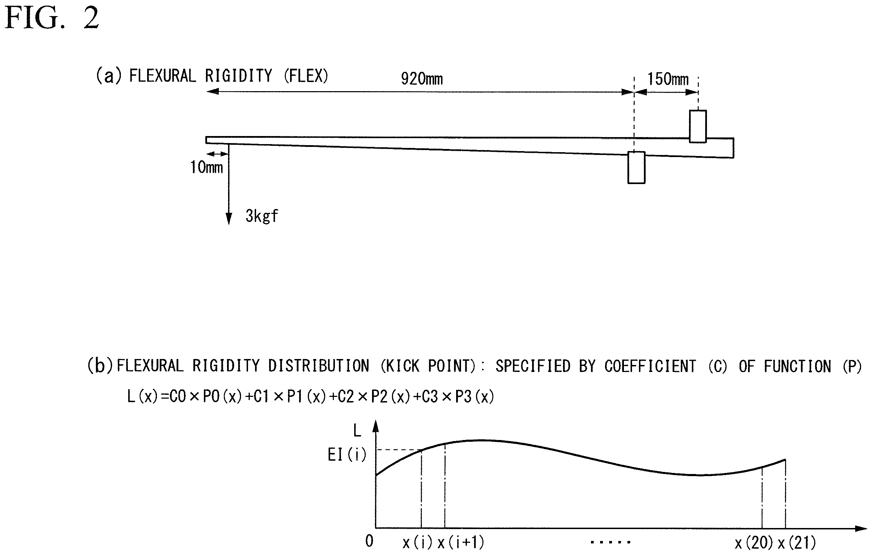

As shown in Fig. 2, three specifications of a shaft selected in the example are the weight of the shaft only, the flex specified by the degree of deflection (Fig. 2(a)), and the kick point specified by coefficient (C) of function (P), indicating the point of maximum bending of the shaft (Fig. 2(b)).

Fig. 2(a) shows an example where a position 920 mm from the tip end of a shaft is supported from below, a position 150 mm further from that point toward the butt end (1070 mm from the tip end) is supported from above, and a load of 3.0 kgf is exerted on a position 10 mm from the tip end. The value of displacement at the tip end indicates the flex (flexural rigidity).

Fig. 2(b) shows examples of kick points (flexural rigidity distribution L (x)). In the graph, "EI (i)" indicates flexural rigidity, and "x" indicates a position on the shaft based on the tip end of the shaft. Flexural rigidity distribution L(x) is represented by a formula $L(x)=C0{\times}P0\,(x)+ C1{\times}P1\,(x)+ C2{\times}P2\,(x)+ C3{\times}P3\,(x).....$

Regarding the point of maximum bending of a shaft, kick points are sorted into a high kick point positioned closer to the grip, a low kick point positioned closer to the head, and a middle kick point positioned in between.

Kick points are categorized as high, low or middle kick points according to the value specified by coefficient (C) of function (P). Swing data are obtained by using one of the aforementioned nine golf clubs 1.

Examples of other specifications applicable to the system are torsional rigidity of a shaft (corresponds to the torque among shaft specifications, hereinafter referred to as "torque"), torsional rigidity distribution of a shaft, shaft weight distribution, golf club length, head weight, club balance, the depth, height and distance of the center of gravity of the head, grip weight, loft angle, lie angle, and face angle.

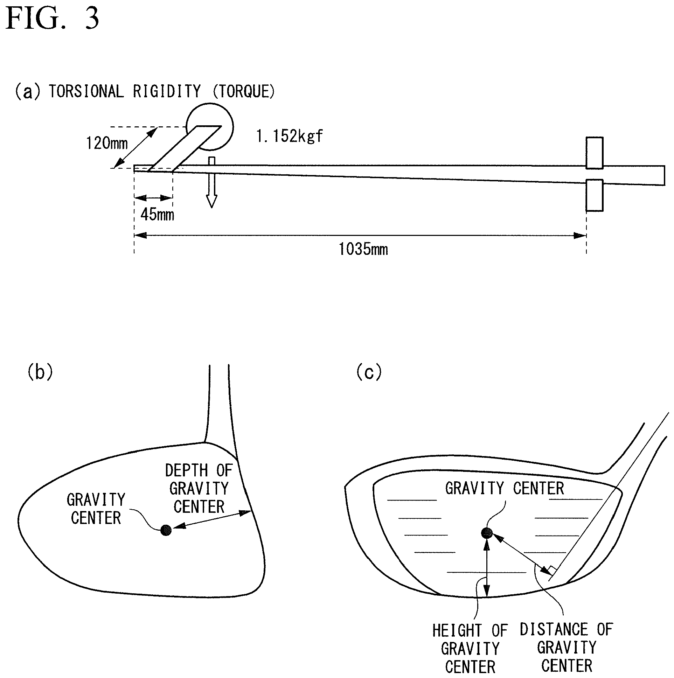

As shown in Fig. 3, the torque is determined by the torsional angle of a shaft. Fig. 3(a) shows an example where a point 1035 mm from the tip end of a shaft is fixed and a torsional rigidity load is exerted on a point 45 mm from the tip of the shaft. Here, (A) is set rotatable while fixed in a direction of bending, and (B) is entirely fixed. In addition, a torsional load of 1.152 kgf is exerted at a point 120 mm from the shaft axis. The torsional angle at the tip end of the shaft obtained above is defined as the torque.

The depth of the center of gravity of the head is specified as the depth (distance) from the face to the center of gravity of a head (Fig. 3(b)). The height of the center of gravity of the head is specified as the length from the leading edge to the center of gravity on the face, and the distance is specified as the length obtained by extending a perpendicular line from the shaft axis toward the center of gravity on the face (Fig. 3(c)). The torsional rigidity distribution and weight distribution of a shaft are described the same as the relationship of the flexural rigidity distribution and kick points.

Club balance (also referred to as swing weight) indicates the feel of the head weight (meaning how the weight of the head feels during a swing or waggle). Club balance is measured by a club balance scale, "Golf Club Scale," made by Kenneth Smith Inc.

The club stiffness is indicated by the frequency of vibration with a grip and head attached thereon. The frequency of vibration is measured by a "Golf Club Timing Harmonizer" made by Fujikura Rubber Ltd. For example, the stiffness of a club is specified as the number of vibrations per minute obtained at a point 760 mm from the grip end when the club vibrates while a point 180 mm from the grip end is fixed.

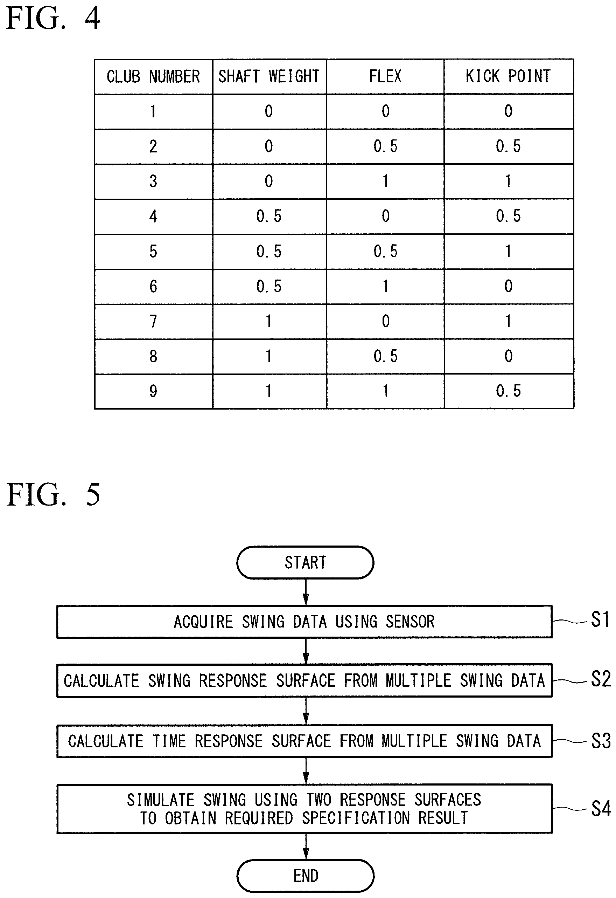

Fig. 4 shows specifications of nine golf clubs for test hitting to obtain swing data based on the L9 orthogonal array in experimental design. Shaft weights show normalized values; the smaller value indicates a greater weight. In addition, flex values are normalized; the smaller value indicates higher rigidity. Shaft weight and flex are both set in a range applicable for a golf shaft when the shaft has a normal length and diameter. Here, regarding the shaft weight, if the level is indicated as "0," the shaft weight is specified as 80g, if it is "0.5," the weight is 70g, and if it is "1," the weight is 60g. Regarding the flex, if the level is indicated as "0," the value of flex (flexural rigidity) is 130 mm, if it is "0.5," the value is 180 mm, and if it is "1," the value is 220 mm. Regarding the kick point, if the level is indicated as "0," it is a low kick point; if it is "0.5," it is a middle kick point; and if it is "1," it is a high kick point. The number of clubs is not limited to nine, and any other number may be tested as long as practical experiments can be conducted.

The heads attached to the golf clubs shown in Fig. 4 are the same, and the shaft length and the weight of each golf club are also the same. Except for the specifications to be analyzed in a fitting process, the rest of the specifications are set the same so that other factors are eliminated from causing variations during the fitting process. In addition, when sensor 11, transmitter 12 and the like are inserted in the shaft, the total weight of sensor 11, transmitter 12 and devices necessary to drive them is set within 20 grams so as to avoid an increase in the entire weight of a golf club 1. Accordingly, by using a commercially available lightweight grip, an increase in the entire weight is suppressed. Thus, negative impacts caused on the swing by an increase in club weight are suppressed.

Next, procedures of golf equipment fitting system 2 shown in Fig. 1 are described with reference to Fig. 5. First, among nine golf clubs 1, a person who performs test hitting (a user: fitting subject) uses a golf club 1 with club No. 1. Sensor 11 outputs the detected results obtained during the test hitting (swing data) to transmitter 12. Transmitter 12 receives the swing data outputted from sensor 11, and transmits the swing data to the outside of the shaft through wireless communication. Receiver 20 receives the swing data and outputs the data to swing data acquisition unit 21. Swing data acquisition unit 21 retains the swing data (step S1).

The above procedure is repeated on other golf clubs 1 (club Nos. 2-9) and swing data acquisition unit 21 retains chronological swing data obtained when the user has conducted at least one test hit on each of nine golf clubs 1. Swing data acquisition unit 21 converts the swing data to moving velocity data of the grip portion of a golf club 1 and the axial rotation data of the shaft. Such conversions are obtained by geometrically calculating the positional relationship between the position of sensor 11 inserted in a golf club 1 and the two points determined in advance on the grip portion of a golf club 1.

In the present embodiment, from the total swing data acquired, swing data acquisition unit 21 converts only the swing data obtained from the position at the top (when the club is in full backswing) to the position at impact (when the head strikes the ball) into moving velocity data and axial rotation data. However, it is an option to set the subject for analysis to be the swing data obtained from address (when the golf club is in contact with the ground) to impact.

Next, swing response surface computation unit 221 reads out the swing data of the nine clubs retained in swing data acquisition unit 21 and calculates a response surface by putting the skills and habits of the user expressed in a linear function (step S2). A swing response surface indicates a relational expression among moving velocity data of the grip portion and axial rotation data obtained during test hitting of the nine golf clubs shown in Fig. 4 and three specifications (shaft weight, flex, kick point) of the golf club.

Formula 1 is as follows. Swing data obtained by multiple golf clubs are represented by $f_1 \sim f_9$, and time (t) is discretized into $t=\{t_1, ..., t_n\}$. Since nine golf clubs are used for test hitting in the present embodiment, swing data is set as $f_1 \sim f_9$. However, the values differ depending on the number of test clubs. Moreover, $f_j(t_i)$ is the measurement value obtained by test hitting No. "j" golf club, more specifically, values of 3-axis acceleration $\{a_x, a_y, a_z\}$ and 3-axis angular velocity $(\omega_x, \omega_y, \omega_z)$.

When three specifications (design parameters) of the nine golf clubs are set as $\{x_j, y_j, z_j\}$ (j=1~9), the relationship represented in formula 1 is obtained. Then, to each "$t_i$", formula 1 is used to obtain solutions.

Here, "x, y, z" are design parameters: "x" is a first specification (shaft weight), "y" is a second specification (flex) and "z" is a third specification (kick point). Numbers "1~n" in "$x_1 \sim x_n$", "$y_1 \sim y_n$" and "$z_1 \sim z_n$" correspond to the club numbers. In addition, any convenient value for analysis is assigned to each of x bar, y bar and z bar (such as intermediate values of design parameters).

[formula 1]

$$\begin{Bmatrix} f_1(t_i) \\ f_2(t_i) \\ \vdots \\ f_9(t_i) \end{Bmatrix} = \begin{bmatrix} 1 & (x_1 - \bar{x}) & (y_1 - \bar{y}) & (z_1 - \bar{z}) \\ 1 & (x_2 - \bar{x}) & (y_2 - \bar{y}) & (z_2 - \bar{z}) \\ & & \vdots & \\ 1 & (x_9 - \bar{x}) & (y_9 - \bar{y}) & (z_9 - \bar{z}) \end{bmatrix} \begin{Bmatrix} a_1(t_i) \\ a_2(t_i) \\ a_3(t_i) \\ a_4(t_i) \end{Bmatrix}$$

By solving formula 1, coefficients "$a_1 \sim a_4$" of the response surface are obtained as expressed by formula 2. When n=5 or greater, formula 1 is typically in excessive conditions, and there is no exact solution available. Thus, a generalized inverse matrix A+ (also referred to as a Moore-Penrose inverse matrix or pseudo-inverse matrix) is used. That is the method for calculating an approximate value when no exact solution is available. Namely, it is a method for obtaining the minimum value of error $|$ Ax-b $|^2$. Since it is a common mathematical method, a detailed description is omitted here. Technical computing software "MATLAB," made by MathWorks Inc., is used to solve formula 1.

Coefficients "$a_1 \sim a_4$" obtained by using formula 2 are the values corresponding to the skills and swing habits of the test hitter. Namely, even when "x, y, z" are changed to specifications that are not measured, swing data represented by function "f" as shown in formula 3 will be obtained. In other words, formula 3 provides approximate values of 3-axis acceleration and 3-axis angular velocity for any parameters {x, y, z}. From formula 3, swing data for No. "m" golf shaft that has not been measured are represented by formula 4. The $f_m(t)$ is a swing response surface obtained by formula 4.

[formula 2]

$$\begin{Bmatrix} a_1(t_i) \\ a_2(t_i) \\ a_3(t_i) \\ a_4(t_i) \end{Bmatrix} = A^+ \begin{Bmatrix} f_1(t_i) \\ f_2(t_i) \\ \\ f_9(t_i) \end{Bmatrix}$$

[formula 3]

$$f(t_i) = a_1(t_i) + a_2(t_i)(x - \bar{x}) + a_3(t_i)(y - \bar{y}) + a_4(t_i)(z - \bar{z})$$

[formula 4]

$$f_m(t) = a_1(t) + a_2(t)(x_m - \bar{x}) + a_3(t)(y_m - \bar{y}) + a_4(t)(z_m - \bar{z})$$

When the swing time varies depending on the golf club and when a swing time for a golf club is set at "$t_k$" (k=1, ..., 9), the sampling time is normalized for a club "k" as represented by formula 5.

[formula 5]

$$t_i = \frac{i}{n} t_k$$

If no significant variation is observed in swing time, the shortest swing time "$t_{min}$" among nine clubs may be employed so that the normalization process is omitted.

Next, time response surface computation unit 222 reads out the swing data of nine clubs retained in swing data acquisition unit 21 and calculates a time response surface with a normalized swing time of the test hitter (step S3). A time response surface means a relational expression among three specifications (here, shaft weight, flex and kick point of a golf club) and swing times obtained when nine golf clubs shown in Fig. 4 were used for test hitting. A time response surface is calculated by using formulas 6~8.

In formula 6, "$g_1 \sim g_9$" indicate swing times of nine golf clubs respectively, and coefficients "$b_1 \sim b_4$" obtained by formula 7 correspond to swing times of the test hitter. By conducting those calculations, the difference in swing times caused by a difference in weights is obtained.

[formula 6]

$$\left\{\begin{matrix} g_1 \\ g_2 \\ \vdots \\ g_9 \end{matrix}\right\} = \begin{bmatrix} 1 & (x_1 - \bar{x}) & (y_1 - \bar{y}) & (z_1 - \bar{z}) \\ 1 & (x_2 - \bar{x}) & (y_2 - \bar{y}) & (z_2 - \bar{z}) \\ & & \vdots & \\ 1 & (x_9 - \bar{x}) & (y_9 - \bar{y}) & (z_9 - \bar{z}) \end{bmatrix} \left\{\begin{matrix} b_1 \\ b_2 \\ b_3 \\ b_4 \end{matrix}\right\}$$

[formula 7]

$$\left\{\begin{matrix} b_1 \\ b_2 \\ b_3 \\ b_4 \end{matrix}\right\} = B^+ \left\{\begin{matrix} g_1 \\ g_2 \\ \vdots \\ g_9 \end{matrix}\right\}$$

Next, each "t" is converted to "$g_m$" based on formula 8. A time response surface is obtained when "$g_m$" is calculated by formula 8. Also, "$f_m'(t)$" is calculated by formula 9 to provide new swing data based on the swing response surface and the time response surface.

[formula 8]

$$g_m = b_1 + b_2(x_m - \bar{x}) + b_3(y_m - \bar{y}) + b_4(z_m - \bar{z})$$

[formula 9]

$$f_m'(t) = f_m\left(\frac{g_m}{t_i n}t\right)$$

Then, the swing data of a golf club that is not used for measurement is calculated by simulation unit 23 based on the moving velocity data and axial rotation data of the grip portion that were measured and retained in swing data acquisition unit 21, the swing response surface calculated by swing response surface computation unit 221 and the time response surface calculated by time response surface computation unit 222. Based on the calculated swing data, simulation unit 23 simulates the movement of a club head using a dynamic finite element method (step S4).

The simulation results showing the movement of a golf club obtained by the above analysis are the club speed at the time of impact, face angle (horizontal angle of the club face relative to the flight line of the ball), and impact loft (loft angle relative to the ground at the time of impact). The club speed corresponds to the carry of the ball, the face angle corresponds to the direction of the ball, and the impact loft corresponds to the height of trajectory. Other than those listed above, the simulation results may be obtained on any other values, for example, club path, attack angle, ball speed, carry and the like.

The analysis of the movement of a club head using a dynamic finite element method is conducted by a known method (a commercially available software for finite element analysis, for example); a detailed description is omitted here. Simulation unit 23 analyzes the movement of a club head by using all the possible specifications of a golf club and by inputting a swing response surface in which the skills and habits of the test hitter are expressed in a linear function and a time response surface in which swing time of the test hitter is normalized. Accordingly, the movement of another golf club is simulated, reflecting the skills, habits and swing time of the test hitter (variations in swing characteristics and swing time using a golf club with different specifications).

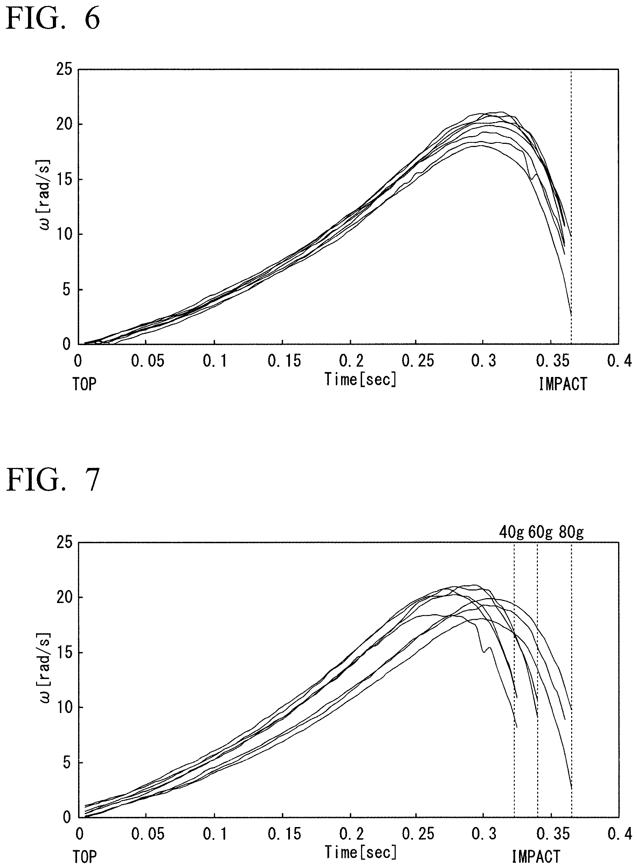

As described above, the weight and length of a golf club have significant impact on the swing time. Figs. 6 and 7 show examples of swing data respectively when shaft weights are the same (Fig. 6) and when shaft weights are different (Fig. 7) ($\omega_x$, for example). When shaft weights are the same, swing times show fewer variations as shown in Fig. 6. On the other hand, when shaft weights differ from each other, for example, 40g, 60g and 80g, swing times vary significantly as shown in Fig. 7.

To solve formula 2 by using a generalized inverse matrix in the above situation, "t" is necessary to be set constant. When swing times do not vary as shown in Fig. 6, no significant error will result when a swing time is set constant by any method. However, when swing times vary notably as shown in Fig. 7, a significantly large error will be caused if swing time is set constant. Thus, time response surface computation unit 222 calculates a time response surface after each "t" is normalized. Then, when "t" is converted again based on the obtained time response surface, swing time data reflecting the swing time are achieved.

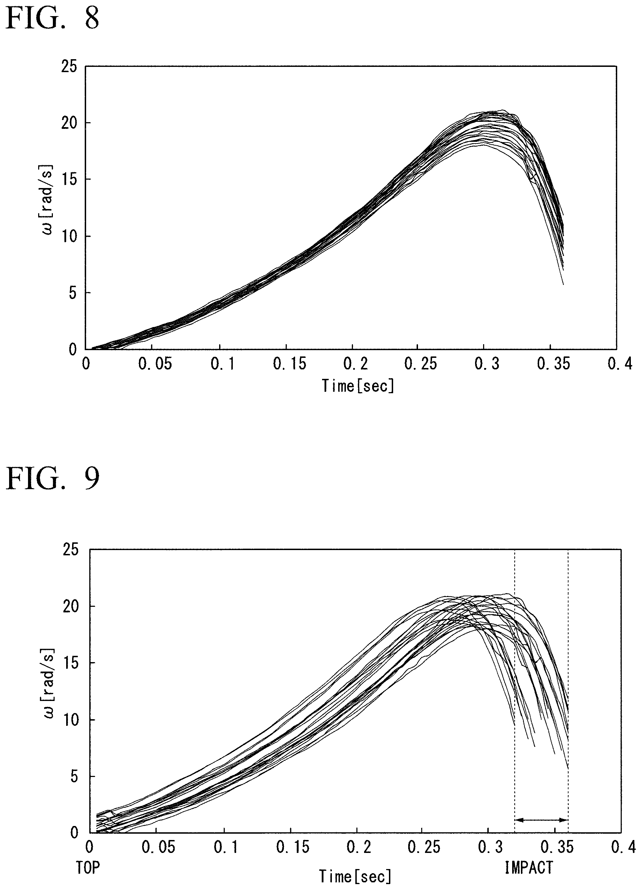

As described above, even when weight and length factors are changed, swing data that reflect a proper swing time are achieved. Thus, accurate simulation results are achieved even on specifications such as weight and length that have significant impact on the swing time. Strictly speaking, swing times also vary on specifications that do not have much impact on the swing time. By introducing a swing response surface and a time response surface, calculation accuracy is improved even on specifications having little impact on swing time. Fig. 8 shows data calculated using only a swing response surface, and Fig. 9 shows data calculated using both swing response surface and time response surface.

Next, a description is provided for the procedures to create result data to be outputted by results output unit 24. Here, regarding the specifications of a shaft to be analyzed, the number of levels of shaft weight (number of levels "i" into which a first specification is divided) is 3, the number of levels of flex (number of levels "j" into which a second specification is divided) is 5, and the number of levels of kick point (number of levels "k" into which a third specification is divided) is 5. The number of levels indicates any selected number of levels for the specifications of nine clubs actually used for test hitting. For example, regarding flex, there are "X, R, L" levels obtained when nine clubs are actually test hit; when the number of levels "j" into which flex is divided is set at 5, two more levels, namely, "S, A" are added to express levels between the three levels (the levels of flex are expressed as "X, S, R, A, L" in order from the stiffest.)

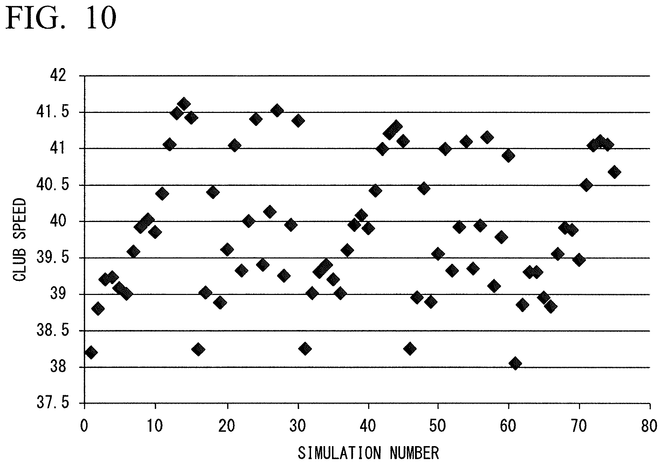

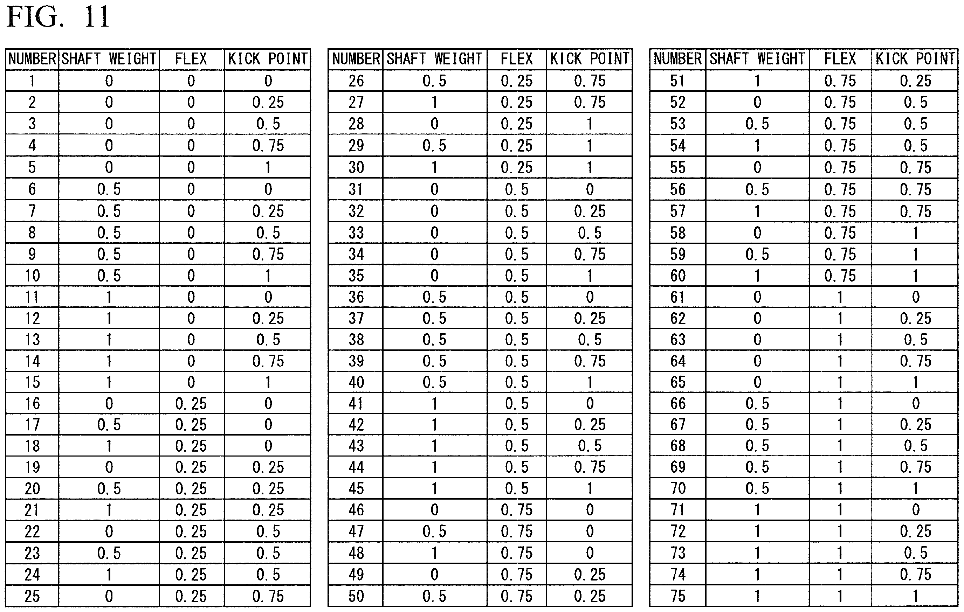

In the above situation, simulation unit 23 obtains simulation results on the movements of golf clubs equipped with $3 \times 5 \times 5 = 75$ shafts. When the result data are outputted for display by entering club speeds on the vertical axis and simulation numbers on the horizontal axis, the display will be as shown in Fig. 10. It is difficult to grasp from Fig. 10 which dot indicates a shaft having a certain specification value. Fig. 11 shows relationships between simulation numbers and club specifications.

Therefore, the following procedure allows a user to find the values of shaft specifications.

First, a result number is allotted to each simulation number based on the following definition. When the number of levels into which a first specification is divided is set as "i" and one level as "l", the number of levels into which a second specification is divided is set as "j" and one level as "m", and the number of levels into which a third specification is divided is set as "k" and one level as "n", then the result number is obtained by (l-1)i + (m-1)j +(n-1)k. In other words, the process is conducted by the following steps.

[1] Setting the lowest level for each of the first and second specifications, the level of the third specification is changed from the lowest to the highest;
[2] Setting the lowest level for the first specification and setting one level higher for the second specification, the level for the third specification is changed from the lowest to the highest;
[3] Step [2] is repeated until the process is conducted for the highest level of the second specification;
[4] Setting one level higher for the first specification, [2] and [3] are conducted; and
[5] Step [4] is repeated until the process is conducted for the highest level of the first specification.

The above procedures are described in detail below:

(A) The shaft weight is fixed to the first level, the flex is fixed to the first level, and the kick point is changed on 5 levels from low kick point toward high kick point;
(B) The shaft weight is fixed to the first level, the flex is fixed to the second level, and the kick point is changed on 5 levels from low kick point toward high kick point;
(C) The shaft weight is fixed to the first level, the flex is fixed to the third level, and the kick point is changed on 5 levels from low kick point toward high kick point;
(D) The shaft weight is fixed to the first level, the flex is fixed to the fourth level, and the kick point is changed on 5 levels from low kick point toward high kick point;
(E) The shaft weight is fixed to the first level, the flex is fixed to the fifth level, and the kick point is changed on 5 levels from low kick point toward high kick point;
(F) The shaft weight is fixed to the second level, the flex is fixed to the first level, and the kick point is changed on 5 levels from low kick point toward high kick point;

(G) The shaft weight is fixed to the second level, the flex is fixed to the second level, and the kick point is changed on 5 levels from low kick point toward high kick point;

(H) The shaft weight is fixed to the second level, the flex is fixed to the third level, and the kick point is changed on 5 levels from low kick point toward high kick point;

(I) The shaft weight is fixed to the second level, the flex is fixed to the fourth level, and the kick point is changed on 5 levels from low kick point toward high kick point;

(J) The shaft weight is fixed to the second level, the flex is fixed to the fifth level, and the kick point is changed on 5 levels from low kick point toward high kick point;

(K) The shaft weight is fixed to the third level, the flex is fixed to the first level, and the kick point is changed on 5 levels from low kick point toward high kick point;

(L) The shaft weight is fixed to the third level, the flex is fixed to the second level, and the kick point is changed on 5 levels from low kick point toward high kick point;

(M) The shaft weight is fixed to the third level, the flex is fixed to the third level, and the kick point is changed on 5 levels from low kick point toward high kick point;

(N) The shaft weight is fixed to the third level, the flex is fixed to the fourth level, and the kick point is changed on 5 levels from low kick point toward high kick point; and

(O) The shaft weight is fixed to the third level, the flex is fixed to the fifth level, and the kick point is changed on 5 levels from low kick point toward high kick point.

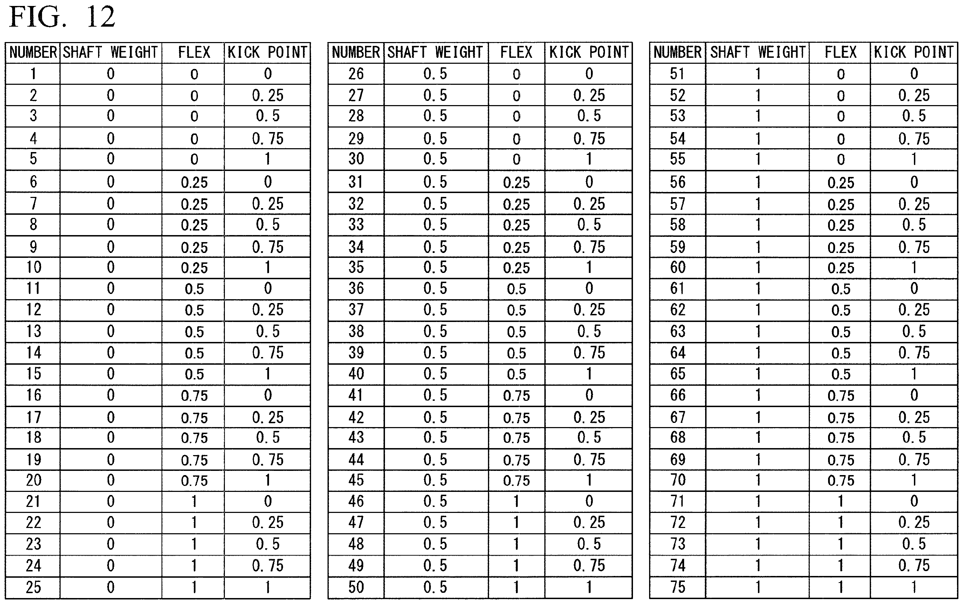

When result numbers are defined in the order of (A)~(O) above, result numbers are determined as shown in Fig. 12. In Fig. 12, the shaft weight "0" means the shaft is heavy (80g, for example), the shaft weight "0.5" means the shaft is midweight (70g, for example), and the shaft weight "1" means the shaft is lightweight (60g, for example). Also, flex "0" means the shaft is stiffest (for example, flex "X"), and as the value increases from flex "0.25" (for example, flex "S"), "0.5" (for example, flex "R") to the "0.75" (for example, flex "A"), it means the shaft is gradually more flexible. Flex "1" indicates the most flexible shaft (for example, flex "L"). Kick point "0" indicates a low kick point, kick point "0.25" indicates a low-mid kick point, kick point "0.5" indicates a middle kick point, kick point "0.75" indicates a mid-high kick point, and kick point "1" indicates a high kick point.

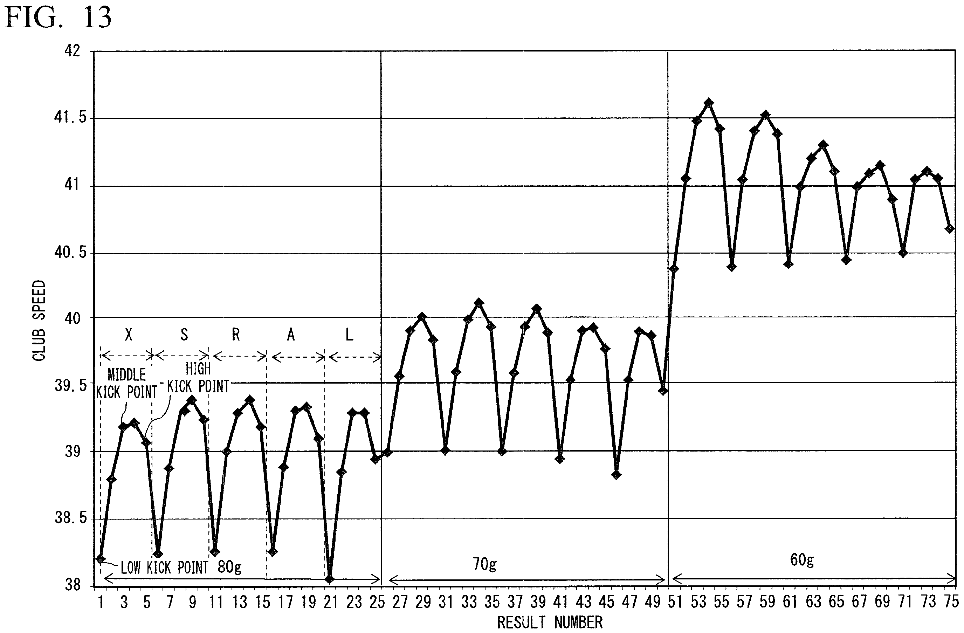

Based on the above, when the obtained values are plotted by entering club speeds on the vertical axis and result numbers on the horizontal axis, Fig. 13 shows the results. When simulation unit 23 conducts simulations in a certain order, and when results output unit 24 outputs the results based on that order, club speed trends are forecast as shown in Fig. 13. However, the outputted pattern as shown in Fig. 13 is still insufficient to provide an easy grasp of the trend.

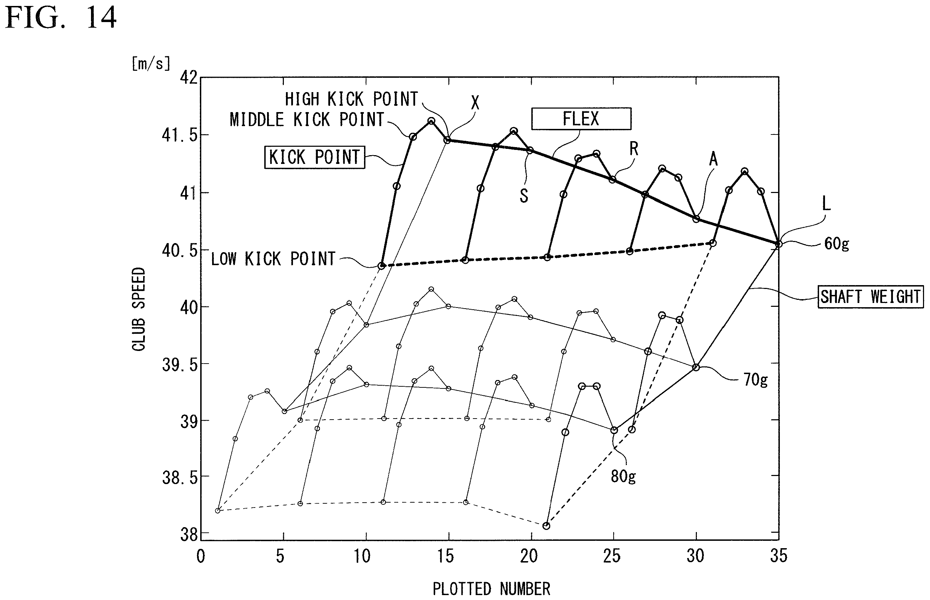

Results output unit 24 subtracts $(j-1) \times k \times (l-1)$ from the result numbers and plots again the obtained values as plotted numbers. Fig. 14 shows fitting results which are obtained by plotting values as above and are outputted accordingly.

More specifically, results output unit 24 assigns plotted numbers by converting result numbers as (a)~(d) below, and outputs the results again by entering the plotted numbers and simulation results, allowing the user to grasp the relationship trend between the specification values of golf equipment and the simulation results.

(a) The results obtained in simulations (A)~(E) are plotted;

(b) The results obtained in simulations (F)~(J) are plotted by shifting the values to the left. The value to be shifted is 20 points on the axis "x" (=75/3-5=20);

(c) The results obtained in simulations (K)~(O) are plotted by shifting the same as above from the values plotted in (b); and

(d) Those that show a difference only in kick points, those that show a difference only in flex levels, and those that show a difference only in shaft weights are connected respectively by straight lines.

To avoid complexity, all kick points are connected, whereas flex levels and shaft weights are connected only at their respective end values of specifications. Here, actual simulation result values are connected by straight lines, but it is an option to approximate points connected by straight lines using a quartic function and to use the obtained approximate values as simulation result values. By using a quartic function approximation method, variations in swings that are likely to occur in a novice golfer are absorbed, and more meaningful results are achieved.

In Fig. 14, the vertical axis shows club speed; a greater value indicates a faster speed (long carry), and a smaller value indicates a slower speed (short carry). The horizontal axis shows newly assigned plotted numbers. In Fig. 14, the mark "○" indicates a golf club that is associated with a specification. Each value is associated with a value of "flex," "kick point" or "shaft weight," which are specifications of the golf shaft.

From the examples of output results shown in Fig. 14, it is found that a shaft weight of "60g," stiffest flex "X" and "mid-high kick point" should be selected to achieve maximum speed. Here, club speed is shown as an example. However, face angle, impact loft and the like are also displayed by using the same method.

Moreover, results output unit 24 may display the trend of specifications by using a natural language. Namely, results output unit 24 may display the fitting results by converting the results into a natural language in association with the corresponding absolute value and inclination for each result. For example, if the purpose of fitting is to increase club speed, results output unit 24 displays "club speed will increase if the flex is stiffer," "club speed will increase if the shaft is lighter," "club speed will increase if the kick point is high," "the impact from the shaft weight is greatest," and the like. If the purpose of fitting is to improve the ball flight by increasing the impact loft, results output unit 24 displays "the ball flight is higher if the flex is stiffer," "the ball flight is higher if the shaft is lighter," "the ball flight is higher if the kick point is low," "the impact of the flex is greatest" and the like. Accordingly, it makes it easier for a user to grasp the trend of specifications by a natural language and to select golf equipment more easily.

Since "club speed" as the purpose of fitting is associated with specifications when the results are outputted, the association with specifications is obtained at a glance. In addition, results output unit 24 is set not only to output the specification of a shaft that exhibits the maximum club speed but also to output all the available specifications. Accordingly, a user can select such specifications that allow the user to achieve a moderate carry, a moderate trajectory height, and a moderate trajectory curve. That is especially effective when face angles are calculated, since it is preferable for the user to select a shaft that exhibits a moderate trajectory curve. When a user intends to produce a trajectory curve and if the user chooses a shaft that causes a maximum curve, the user may face an excessive trajectory curve.

Fig. 14 shows examples where results output unit 24 displays information using characters associated with specifications. However, related specifications may also be displayed in different colors. To make the display easier to grasp, results output unit 24 may output the results by changing the color density of the lines or the like so that those including the best values will stand out. Moreover, as further simplified data of those shown in Fig. 14, results output unit 24 may output another communication method that replaces a natural language.

In the above descriptions, the shaft weight (3 levels) is set as a first specification, the flex (5 levels) is set as a second specification, and the kick point (5 levels) is set as a third specification. However, that is not the only option.

For example, it is an option to set the torque (5 levels) as a first specification, the flex (5 levels) as a second specification, and the kick point (5 levels) as a third specification. Alternatively, it is another option to set the flex (5 levels) as a first specification, the torque (5 levels) as a second specification, and the shaft weight (3 levels) as a third specification.

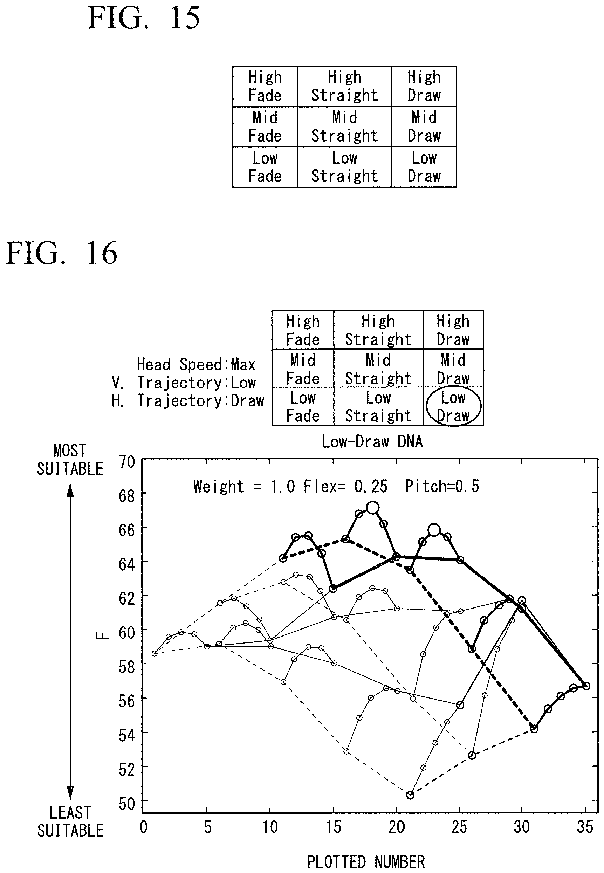

Next, procedures are described to output complex conditions such as club speed and trajectory height, or club speed and trajectory curve, as fitting results. Generally, a golfer requires for a golf club not a single performance but complex performances; for example, preventing slice, while maximizing club speed and simultaneously increasing trajectory height, or the like. When complex conditions are outputted, one of the nine trajectories shown in Fig. 15 is selected by a user. The trajectory of a ball is determined by the height and direction of the trajectory. When trajectory height is sorted into "High" (high trajectory), "Mid" (medium trajectory) and "Low" (low trajectory), and the trajectory direction is sorted into "Fade," "Straight" and "Draw," nine combinations are obtained as shown in Fig. 15. Here, "Fade" means the trajectory curves to the right when a user is right-handed, and "Draw" means the trajectory curves to the left when a user is right-handed. Then, results output unit 24 outputs conditions that satisfy the selected trajectory while the club speed is maximized. To output complex conditions, specifications that maximize a target function (F) are selected. Target function (F) is expressed in "$F = \alpha \times f_1 + \beta \times f_2 + \gamma \times f_3$."

In the above formula, "$f_1$" represents first result data (club speed, for example), "$f_2$" represents second result data (face angle, for example), "$f_3$" represents third result data (impact loft, for example), and "$\alpha, \beta, \gamma$" are each a weight coefficient. Here, "$\alpha, \beta, \gamma$" are selected properly according to the choice made by a user. Generally, "$\alpha$" is preferred to be 1-3 times the value of "$\beta + \gamma$." That is because club speed is most important for a golfer.

Fig. 16 shows examples of fitting results when a Low/Draw trajectory and maximum club speed are set as complex conditions. Here, the vertical axis shows which shaft is the best to hit a Low/Draw trajectory while increasing club speed. From the graph, the best specifications for the user are the shaft weight of 60g, flex of "S" and middle kick point.

Results output unit 24 may display the specification that shows the maximum simulation results (club speed, impact loft, face angle and the like) by converting the results into actual product names of golf equipment.

As described, by selecting a trajectory, a user can select a shaft most preferable to achieve the selected trajectory. Accordingly, the user can easily select the golf equipment most suitable to achieve desired trajectory. Namely, golf equipment fitting is conducted by the aforementioned golf equipment fitting system. The system is capable of conducting measurement and analysis, and displaying the results in a short period of time. Accordingly, the system provides a user with visual determination for specifications of the most suitable golf shaft.

In the example above, the shaft weight, flex and kick point are selected as factors. However, that is not the only option. Also, factors are not limited to 3 specifications, and more or fewer specifications may be selected. As described above, it is preferred to select specifications that may affect swing time (shaft weight and club length, for example). Other specifications to be selected are the flexural rigidity, torsional rigidity, weight, flexural rigidity distribution, torsional rigidity distribution and weight distribution of a shaft; the length of a golf club; head weight; club balance; the depth, height and distance of the center of gravity of the head; grip weight; loft angle, lie angle and face angle.

As for a time response surface, the data with the longest swing time may be used so as to set all the swing times to be equal. In such a case, moderate accuracy and shortened calculation time are achieved.

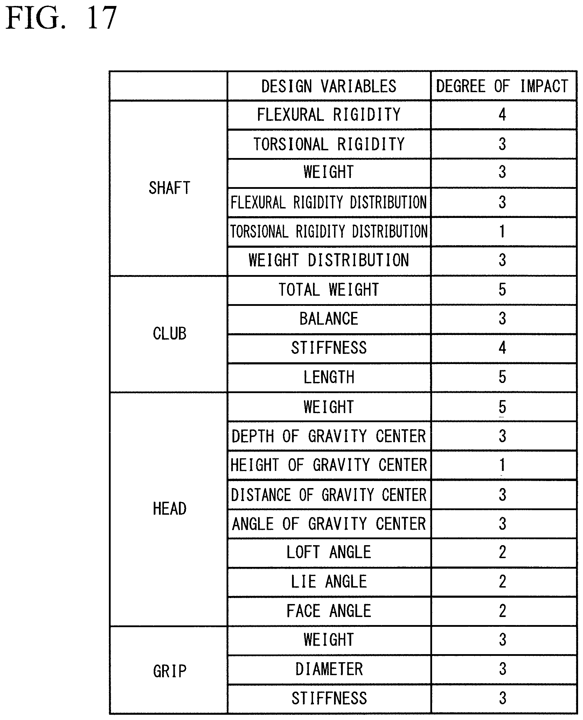

When multiple different specifications are selected, it is preferred to select a combination of those having similar impact degrees. An impact degree means the degree of impact on the final behavior of the head when certain specifications are changed. Fig. 17 shows degrees of impact. The reasons are as follows.

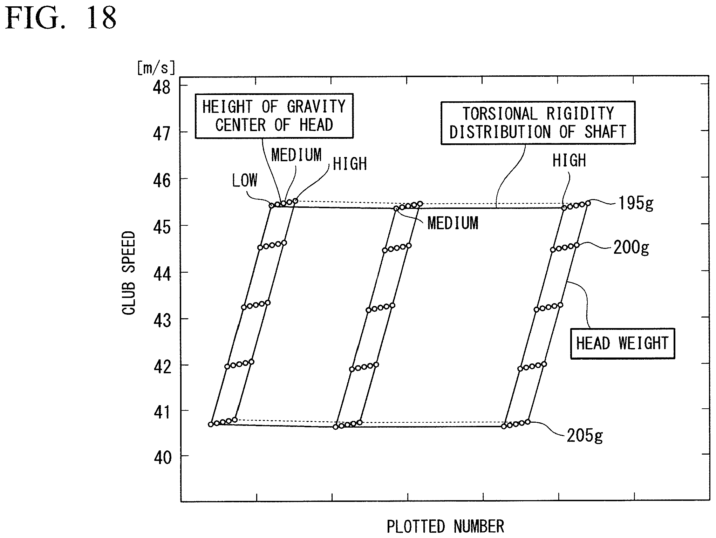

Fig. 18 shows the result of simulations when three different specifications are as follows: head weight (impact degree: 5), torsional rigidity distribution of a shaft (impact degree: 1), and height of the center of gravity of the head (impact degree: 1). When specifications have impact degrees significantly different from each other, the obtained data mostly display the specifications with a greater impact degree, and the results of the rest with smaller impact degrees are hard to read. Thus, it is preferred to select specifications having impact degrees within a certain range (in the present embodiment, within 2). Also, the impact degrees of three different specifications are more preferred to be the same. In the example shown in Fig. 18, it is found that head speed varies depending on the head weight, but no difference is found in the two other specifications.

When three specifications are combined, it is preferred to use shaft weight, flexural rigidity (flex) and flexural rigidity distribution (kick point). In such a combination, the results are preferably used for fitting and designing shafts.

In addition, flexural rigidity (flex), club length and head weight may be combined. In such a combination, the results are preferably used for fitting and designing clubs.

Moreover, the height, depth, distance and angle of the center of gravity of the head may be combined. In such a combination, the results are preferably used for fitting and designing heads.

Furthermore, the loft angle, lie angle and face angle of a head may be combined. In such a combination, the results are preferably used for fitting and designing heads. Also, the stiffness, length, total weight and balance of a club may be combined.

Preferred combinations are listed above. However, they are not the only options.

The program for executing functions of the golf equipment fitting system may be recorded on a computer readable memory medium, and a computer system may read out the stored program on the medium and execute the program. Here, "a computer system reads out the stored program on a recording medium and executes the program" includes installing the program in a computer system. Here, a "computer system" includes the OS and hardware such as peripheral devices. Also, a "computer system" may include multiple computer devices connected through the Internet, WAN, LAN and networks that include exclusive communication lines. "Computer readable recording media" means portable media such as flexible disks, magneto-optical disks, ROM and CD-ROM, and recording devices such as hard discs built into a computer system. The recording medium to store the program may be a non-transitory recoding medium such as a CD-ROM. Also, a recording medium includes a memory medium installed internally or externally to be accessible for a distribution server to distribute the program. The code stored in the recording medium of the distribution server may be different from the code that allows the terminal device to execute the program. Namely, the memory code to be stored in a distribution server is not limited specifically as long as it allows the terminal device to download and install the program from the distribution server so that the program is executed. In addition, the program may be divided into multiple sections and integrated back in a terminal device after the sections are downloaded at different times, or distribution servers for each divided section may be different. Furthermore, "computer readable recording media" includes a medium for retaining the program for a certain duration such as volatile memory (RAM) inside a computer system that becomes a server or a client when the program is transmitted through networks. In addition, the above program may be intended to implement part of the functions described above. Alternatively, the above program may be a so-called differential file (differential program) for implementing the above functions in combination with another program already installed in the computer system.

Also, some or all of the above functions may be implemented as an integrated circuit such as LSI (large scale integration). The functions may be set individually in separate processors. Some or all of the above functions may be integrated and set in a processor. To integrate the functions, LSIs, or exclusive or generic processors may be used. Also, when new integration technology is made available to replace LSIs because of semiconductor technological development, integrated circuits by such technology may also be applied.

INDUSTRIAL APPLICABILITY

As described above, the golf equipment fitting system and golf equipment fitting program related to the present invention are capable of fitting golf equipment according to the desires, skills and habits of a golfer.

[DESCRIPTION OF NUMERICAL REFERENCES]

1... golf club, 11...sensor, 12... transmitter, 2... golf shaft fitting system, 20... receiver unit, 21... swing data acquisition unit, 221... swing response surface computation unit, 222...time response surface computation unit, 23... simulation unit, 24... results output unit

**Claims**

1. A golf equipment fitting system, comprising:

   a swing data acquisition unit (21) that acquires swing data from a sensor (11) attached to a plurality of golf clubs with different specifications;
   a swing response surface computation unit (221) that calculates a swing response surface using the response surface methodology based on the swing data obtained by the swing data acquisition unit (21);
   **characterized by**
   a time response surface computation unit (222) that calculates a time response surface using the response surface methodology based on the swing data obtained by the swing data acquisition unit (21);
   a simulation unit (23) that calculates the swing data of a golf club that is not used for measurement from the swing response surface and the time response surface and simulates the swing of the golf club based on the obtained swing data; and
   a results output unit (24) that outputs the results of simulations conducted by the simulation unit (23).

2. The golf equipment fitting system according to Claim 1, wherein the specifications of a plurality of golf clubs to be used in simulations include at least the weight of a shaft.

3. The golf equipment fitting system according to Claim 1 or 2, wherein the specifications of a plurality of golf clubs to be used in simulations include at least the length of a club.

4. The golf equipment fitting system according to any of Claims 1~3, wherein the specifications of a plurality of golf clubs to be used in simulations include at least two different specifications, and the difference in impact degrees of the two different specifications is within a predetermined value.

5. The golf equipment fitting system according to any of Claims 1-4, wherein the specifications of a plurality of golf clubs to be used in simulations are nine specifications selected from among 27 specifications obtained in combination of three different specifications and three levels each of the three specifications based on an L9 orthogonal array for the design of experiments.

6. The golf equipment fitting system according to Claim 5, wherein the three different specifications are shaft weight, kick point and flex.

7. The golf equipment fitting system according to any of Claims 1~4, wherein the three different specifications are selected from among the stiffness, length, total weight and balance of a club.

8. The golf equipment fitting system according to any of Claims 1~4, wherein the three different specifications are selected from among the height, depth, distance and angle of the center of gravity of the head.

9. The golf equipment fitting system according to any of Claims 1~4, wherein the three different specifications are the loft angle, lie angle and face angle of a club head.

10. The golf equipment fitting system according to any of Claims 5~9, wherein when the three different specifications are divided into their respective levels and when the number of levels of a first specification is set as (i) and one level as (1), the number of levels of a second specification is set as (j) and one level as (m), and the number of levels of a third specification is set as (k) and one level as (n), the results output unit (24) allocates a result number defined by (l-1)i+(m-1)j+(n-1)k to each of the simulation results, then, each result number is replaced with a plotted number, which is the value obtained by subtracting $(j-1)\times k\times(l-1)$ from the result number, and finally the results output unit (24) outputs data as the fitting results by entering the plotted numbers on the horizontal axis and the simulation results on the vertical axis.

**11.** The golf equipment fitting system according to Claim 10, wherein the results output unit (24) outputs the fitting results as line segments by connecting the plotted data applicable to the following conditions (i)~(iii):

(i) data in which a first specification is different from second and third specifications while second and third specifications are the same;
(ii) data in which a second specification is different from first and third specifications while first and third specifications are the same; and
(iii) data in which a third specification is different from first and second specifications while first and second specifications are the same.

**12.** The golf equipment fitting system according to Claim 10 or 11, wherein the results output unit (24) outputs fitting results by converting the results to a natural language in association with corresponding absolute values and inclinations.

**13.** The golf equipment fitting system according to any of Claims 1~4, wherein the results output unit (24) outputs a specification that exhibits the maximum value in simulation results by converting the specification to a product name.

**14.** The golf equipment fitting system according to Claim 1, wherein the results output unit (24) selects and displays a golf club based on the results of simulations conducted by the simulation unit (23).

**15.** A golf equipment fitting program to be implemented by a computer, comprising:

a swing data acquisition step to acquire swing data by attaching a sensor (11) to a plurality of golf clubs with different specifications;
a swing response surface computation step to calculate a swing response surface using the response surface methodology based on the swing data obtained by the swing data acquisition step;
**characterized by**
a time response surface computation step to calculate a time response surface using the response surface methodology based on the swing data obtained by the swing data acquisition step;
a simulation step to calculate the swing data of a golf club that is not used for measurement from the swing response surface and the time response surface and to simulate the swing of the golf club based on the obtained swing; and
a results outputting step to output the results of simulations conducted by the simulation step.

**Patentansprüche**

**1.** Passsystem für Golfvorrichtung, mit:

einer Schwungdaten-Erfassungseinheit (21), die Schwungdaten von einem Sensor (11) erfasst, der an einer Mehrzahl von Golfschlägern mit verschiedenen Spezifikationen angebracht ist;
einer Schwung-Antwortflächen-Berechnungseinheit (221), die eine Schwung-Antwortfläche unter Verwendung der Antwortflächen-Methode basierend auf den durch die Schwungdaten-Erfassungseinheit (21) erhaltenen Schwungdaten berechnet;
**gekennzeichnet durch**
eine Zeit-Antwortflächen-Berechnungseinheit (222), die eine Zeit-Antwortfläche unter Verwendung der Antwortflächen-Methode basierend auf den **durch** die Schwungdaten-Erfassungseinheit (21) erhaltenen Schwungdaten berechnet;
eine Simulationseinheit (23), die die Schwungdaten von einem Golfschläger, der nicht zur Messung verwendet wird, aus der Schwung-Antwortfläche und der Zeit-Antwortfläche berechnet und den Schwung des Golfschlägers basierend auf den erhaltenen Schwungdaten simuliert; und
eine Ergebnis-Ausgabeeinheit (24), die die Ergebnisse von **durch** die Simulationseinheit (23) durchgeführten Simulationen ausgibt.

**2.** Passsystem für Golfvorrichtung nach Anspruch 1, wobei die Spezifikationen einer Mehrzahl von in Simulationen zu verwendenden Golfschlägern mindestens das Gewicht eines Schafts beinhalten.

**3.** Passsystem für Golfvorrichtung nach Anspruch 1 oder 2, wobei die Spezifikationen einer Mehrzahl von in Simulationen zu verwendenden Golfschlägern mindestens die Länge eines Schlägers beinhalten.

4. Passsystem für Golfvorrichtung nach einem der Ansprüche 1-3, wobei die Spezifikationen einer Mehrzahl von in Simulationen zu verwendenden Golfschlägern mindestens zwei verschiedene Spezifikationen beinhalten, und der Unterschied in den Auswirkungsgraden der zwei verschiedenen Spezifikationen innerhalb eines vorbestimmten Werts liegt.

5. Passsystem für Golfvorrichtung nach einem der Ansprüche 1-4, wobei die Spezifikationen einer Mehrzahl von in Simulationen zu verwendenden Golfschlägern neun Spezifikationen sind, ausgewählt aus 27 Spezifikationen, die in Kombination von drei verschiedenen Spezifikationen und drei Stufen erhalten werden, wobei jede der drei Spezifikationen auf einem L9-orthogonalem Feld für den Versuchsplan basiert.

6. Passsystem für Golfvorrichtung nach Anspruch 5, wobei die drei verschiedenen Spezifikationen Schaftgewicht, Kick-Point und Biegsamkeit sind.

7. Passsystem für Golfvorrichtung nach einem der Ansprüche 1-4, wobei die drei verschiedenen Spezifikationen aus Steifigkeit, Länge, Gesamtgewicht und Balance eines Schlägers ausgewählt sind.

8. Passsystem für Golfvorrichtung nach einem der Ansprüche 1-4, wobei die drei verschiedenen Spezifikationen aus Höhe, Tiefe, Abstand und Winkel des Schwerpunkts des Kopfes ausgewählt sind.

9. Passsystem für Golfvorrichtung nach einem der Ansprüche 1-4, wobei die drei verschiedenen Spezifikationen der Loft-Winkel, Lie-Winkel und Face-Winkel eines Schlägerkopfes sind.

10. Passsystem für Golfvorrichtung nach einem der Ansprüche 5-9, wobei wenn die drei verschiedenen Spezifikationen in ihre jeweiligen Stufen eingeteilt werden und wenn die Zahl von Stufen einer ersten Spezifikation als (i) und eine Stufe als (l) festgelegt werden, die Zahl von Stufen einer zweiten Spezifikation als (j) und eine Stufe als (m) festgelegt werden, und die Zahl von Stufen einer dritten Spezifikation als (k) und eine Stufe als (n) festgelegt werden, die Ergebnis-Ausgabeeinheit (24) jedem der Simulationsergebnisse eine Ergebniszahl zuweist, die durch $(l-1)i+(m-1)j+(n-1)k$ definiert ist, dann jede Ergebniszahl mit einer aufgetragenen Zahl ersetzt wird, die der Wert ist, der durch Subtrahieren von $(j-1) \times k \times (l-1)$ von der Ergebniszahl erhalten wird, und schließlich die Ergebnis-Ausgabeeinheit (24) Daten als die Passergebnisse durch Eintragen der aufgetragenen Zahlen auf der horizontalen Achse und der Simulationsergebnisse auf der vertikalen Achse ausgibt.

11. Passsystem für Golfvorrichtung nach Anspruch 10, wobei die Ergebnis-Ausgabeeinheit (24) die Passergebnisse als Liniensegmente ausgibt durch Verbinden der aufgetragenen Daten, auf die folgende Bedingungen (i)-(iii) anwendbar sind:

(i) Daten, bei denen eine erste Spezifikation von zweiten und dritten Spezifikationen verschieden ist, während zweite und dritte Spezifikationen gleich sind;
(ii) Daten, bei denen eine zweite Spezifikation von ersten und dritten Spezifikationen verschieden ist, während erste und dritte Spezifikationen gleich sind; und
(iii) Daten, bei denen eine dritte Spezifikation von ersten und zweiten Spezifikationen verschieden ist, während erste und zweite Spezifikationen gleich sind.

12. Passsystem für Golfvorrichtung nach Anspruch 10 oder 11, wobei die Ergebnis-Ausgabeeinheit (24) Passergebnisse durch Konvertieren der Ergebnisse in eine natürliche Sprache in Verbindung mit entsprechenden absoluten Werten und Neigungen ausgibt.

13. Passsystem für Golfvorrichtung nach einem der Ansprüche 1-4, wobei die Ergebnis-Ausgabeeinheit (24) eine Spezifikation ausgibt, die den maximalen Wert in Simulationsergebnissen durch Konvertieren der Spezifikation in einen Produktnamen zeigt.

14. Passsystem für Golfvorrichtung nach Anspruch 1, wobei die Ergebnis-Ausgabeeinheit (24) einen Golfschläger basierend auf den Ergebnissen von durch die Simulationseinheit (23) durchgeführten Simulationen auswählt und anzeigt.

15. Passprogramm für Golfvorrichtung, das von einem Computer zu implementieren ist, mit:

einem Schwungdaten-Erfassungsschritt zum Erfassen von Schwungdaten durch Anbringen eines Sensors (11) an einer Mehrzahl von Golfschlägern mit verschiedenen Spezifikationen;

einem Schwung-Antwortflächen-Berechnungsschritt zum Berechnen einer Schwung-Antwortfläche unter Verwendung der Antwortflächen-Methode basierend auf den durch den Schwungdaten-Erfassungsschritt erhaltenen Schwungdaten;

**gekennzeichnet durch**

einen Zeit-Antwortflächen-Berechnungsschritt zum Berechnen einer Zeit-Antwortfläche unter Verwendung der Antwortflächen-Methode basierend auf den durch den Schwungdaten-Erfassungsschritt erhaltenen Schwungdaten;

einen Simulationsschritt zum Berechnen der Schwungdaten eines Golfschlägers, der nicht zur Messung verwendet wird, aus der Schwung-Antwortfläche und der Zeit-Antwortfläche und zum Simulieren des Schwungs des Golfschlägers basierend auf dem erhaltenen Schwung; und

einen Ergebnis-Ausgabeschritt zum Ausgeben der Ergebnisse von **durch** den Simulationsschritt durchgeführten Simulationen.

## Revendications

1. Système d'ajustement d'équipement de golf, comprenant :

une unité d'acquisition de données de swing (21) qui acquiert des données de swing en provenance d'un capteur (11) attaché à une pluralité de clubs de golf avec différentes spécifications ;
une unité de calcul de surface de réponse de swing (221) qui calcule une surface de réponse de swing en utilisant la méthodologie de surface de réponse sur la base des données de swing obtenues par l'unité d'acquisition de données de swing (21) ;
**caractérisé par**
une unité de calcul de surface de réponse de temps (222) qui calcule une surface de réponse de temps en utilisant la méthodologie de surface de réponse sur la base des données de swing obtenues par l'unité d'acquisition de données de swing (21) ;
une unité de simulation (23) qui calcule les données de swing d'un club de golf qui n'est pas utilisé pour la mesure à partir de la surface de réponse de swing et de la surface de réponse de temps et simule le swing du club de golf sur la base des données de swing obtenues; et
une unité de production de résultats (24) qui produit les résultats de simulations effectuées par l'unité de simulation (23).

2. Système d'ajustement d'équipement de golf selon la revendication 1, dans lequel les spécifications d'une pluralité de clubs de golf devant être utilisés dans des simulations incluent au moins le poids d'un manche.

3. Système d'ajustement d'équipement de golf selon la revendication 1 ou 2, dans lequel les spécifications d'une pluralité de clubs de golf devant être utilisés dans des simulations incluent au moins la longueur d'un club.

4. Système d'ajustement d'équipement de golf selon l'une quelconque des revendications 1-3, dans lequel les spécifications d'une pluralité de clubs de golf devant être utilisés dans des simulations incluent au moins deux spécifications différentes, et la différence en degrés d'impact des deux spécifications différentes est à l'intérieur d'une valeur prédéterminée.

5. Système d'ajustement d'équipement de golf selon l'une quelconque des revendications 1-4, dans lequel les spécifications d'une pluralité de clubs de golf devant être utilisés dans des simulations sont neuf spécifications sélectionnées parmi 27 spécifications obtenues en combinaison de trois spécifications différentes et trois niveaux de chacune des trois spécifications sur la base d'un réseau L9 orthogonal pour la conception d'expériences.

6. Système d'ajustement d'équipement de golf selon la revendication 5, dans lequel les trois spécifications différentes sont le poids du manche, le point de coup et la flexion.

7. Système d'ajustement d'équipement de golf selon l'une quelconque des revendications 1-4, dans lequel les trois spécifications différentes sont sélectionnées parmi la rigidité, la longueur, le poids total et l'équilibre d'un club.

8. Système d'ajustement d'équipement de golf selon l'une quelconque des revendications 1-4, dans lequel les trois spécifications différentes sont sélectionnées parmi la hauteur, la profondeur, la distance et l'angle du centre de gravité de la tête.

9. Système d'ajustement d'équipement de golf selon l'une quelconque des revendications 1-4, dans lequel les trois spécifications différentes sont l'angle d'ouverture, l'angle de repos et l'angle de face d'une tête de club.

10. Système d'ajustement d'équipement de golf selon l'une quelconque des revendications 5-9, dans lequel lorsque les trois spécifications différentes sont divisées en leurs niveaux respectifs et lorsque le nombre de niveaux d'une première spécification est défini en tant que (i) et un niveau en tant que (l), le nombre de niveaux d'une deuxième spécification est défini en tant que (j) et un niveau en tant que (m), et le nombre de niveaux d'une troisième spécification est défini en tant que (k) et un niveau en tant que (n), l'unité de production de résultats (24) attribue un nombre de résultat défini par (l-1)i+(m-1)j+(n-1)k à chacun des résultats de simulation, ensuite, chaque nombre de résultat est remplacé par un nombre tracé, qui est la valeur obtenue en soustrayant (j-1) x k x (l-1) du nombre de résultat, et finalement l'unité de production de résultats (24) produit des données en tant que résultats d'ajustement en saisissant les nombres tracés sur l'axe horizontal et les résultats de simulation sur l'axe vertical.

11. Système d'ajustement d'équipement de golf selon la revendication 10, dans lequel l'unité de production de résultats (24) produit les résultats d'ajustement en tant que segments de ligne en reliant les données tracées applicables aux conditions suivantes (i)-(iii) :

   (i) des données dans lesquelles une première spécification est différente des deuxième et troisième spécifications pendant que les deuxième et troisième spécifications sont les mêmes ;
   (ii) des données dans lesquelles une deuxième spécification est différente des première et troisième spécifications pendant que les première et troisième spécifications sont les mêmes ; et
   (iii) des données dans lesquelles une troisième spécification est différente des première et deuxième spécifications pendant que les première et deuxième spécifications sont les mêmes.

12. Système d'ajustement d'équipement de golf selon la revendication 10 ou 11, dans lequel l'unité de production de résultats (24) produit des résultats d'ajustement en convertissant les résultats en un langage naturel en association avec des valeurs absolues correspondantes et des inclinaisons.

13. Système d'ajustement d'équipement de golf selon l'une quelconque des revendications 1-4, dans lequel l'unité de production de résultats (24) produit une spécification qui expose la valeur maximale dans des résultats de simulation en convertissant la spécification en un nom de produit.

14. Système d'ajustement d'équipement de golf selon la revendication 1, dans lequel l'unité de production de résultats (24) sélectionne et affiche un club de golf sur la base des résultats des simulations effectuées par l'unité de simulation (23).

15. Programme d'ajustement d'équipement de golf devant être mis en oeuvre par un ordinateur, comprenant :

   une étape d'acquisition de données de swing pour acquérir des données de swing en attachant un capteur (11) à une pluralité de clubs de golf ayant différentes spécifications ;
   une étape de calcul de surface de réponse de swing pour calculer une surface de réponse de swing en utilisant la méthodologie de surface de réponse sur la base des données de swing obtenues par l'étape d'acquisition de données de swing;
   **caractérisé par**
   une étape de calcul de surface de réponse de temps pour calculer une surface de réponse de temps en utilisant la méthodologie de surface de réponse sur la base des données de swing obtenues par l'étape d'acquisition de données de swing ;
   une étape de simulation pour calculer les données de swing d'un club de golf qui n'est pas utilisé pour la mesure à partir de la surface de réponse de swing et de la surface de réponse de temps et pour simuler le swing du club de golf sur la base du swing obtenu ; et
   une étape de production de résultats pour produire les résultats des simulations effectuées par l'étape de simulation.

FIG. 1

EP 2 962 741 B1

# FIG. 2

(a) FLEXURAL RIGIDITY (FLEX)

150mm

920mm

10mm

3kgf

(b) FLEXURAL RIGIDITY DISTRIBUTION (KICK POINT) : SPECIFIED BY COEFFICIENT (C) OF FUNCTION (P)

$L(x) = C0 \times P0(x) + C1 \times P1(x) + C2 \times P2(x) + C3 \times P3(x)$

L

EI(i)

0    x(i) x(i+1)    x(20) x(21)

# FIG. 3

(a) TORSIONAL RIGIDITY (TORQUE)

120mm

1.152kgf

45mm

1035mm

(b)

DEPTH OF
GRAVITY
CENTER

GRAVITY
CENTER

(c)

GRAVITY CENTER

HEIGHT OF
GRAVITY
CENTER

DISTANCE OF
GRAVITY
CENTER

# FIG. 4

| CLUB NUMBER | SHAFT WEIGHT | FLEX | KICK POINT |
|---|---|---|---|
| 1 | 0 | 0 | 0 |
| 2 | 0 | 0.5 | 0.5 |
| 3 | 0 | 1 | 1 |
| 4 | 0.5 | 0 | 0.5 |
| 5 | 0.5 | 0.5 | 1 |
| 6 | 0.5 | 1 | 0 |
| 7 | 1 | 0 | 1 |
| 8 | 1 | 0.5 | 0 |
| 9 | 1 | 1 | 0.5 |

# FIG. 5

START

ACQUIRE SWING DATA USING SENSOR — S1

CALCULATE SWING RESPONSE SURFACE FROM MULTIPLE SWING DATA — S2

CALCULATE TIME RESPONSE SURFACE FROM MULTIPLE SWING DATA — S3

SIMULATE SWING USING TWO RESPONSE SURFACES
TO OBTAIN REQUIRED SPECIFICATION RESULT — S4

END

FIG. 6

FIG. 7

FIG. 8

FIG. 9

FIG. 10

FIG. 11

| NUMBER | SHAFT WEIGHT | FLEX | KICK POINT | NUMBER | SHAFT WEIGHT | FLEX | KICK POINT | NUMBER | SHAFT WEIGHT | FLEX | KICK POINT |
|---|---|---|---|---|---|---|---|---|---|---|---|
| 1 | 0 | 0 | 0 | 26 | 0.5 | 0.25 | 0.75 | 51 | 1 | 0.75 | 0.25 |
| 2 | 0 | 0 | 0.25 | 27 | 1 | 0.25 | 0.75 | 52 | 0 | 0.75 | 0.5 |
| 3 | 0 | 0 | 0.5 | 28 | 0 | 0.25 | 1 | 53 | 0.5 | 0.75 | 0.5 |
| 4 | 0 | 0 | 0.75 | 29 | 0.5 | 0.25 | 1 | 54 | 1 | 0.75 | 0.5 |
| 5 | 0 | 0 | 1 | 30 | 1 | 0.25 | 1 | 55 | 0 | 0.75 | 0.75 |
| 6 | 0.5 | 0 | 0 | 31 | 0 | 0.5 | 0 | 56 | 0.5 | 0.75 | 0.75 |
| 7 | 0.5 | 0 | 0.25 | 32 | 0 | 0.5 | 0.25 | 57 | 1 | 0.75 | 0.75 |
| 8 | 0.5 | 0 | 0.5 | 33 | 0 | 0.5 | 0.5 | 58 | 0 | 0.75 | 1 |
| 9 | 0.5 | 0 | 0.75 | 34 | 0 | 0.5 | 0.75 | 59 | 0.5 | 0.75 | 1 |
| 10 | 0.5 | 0 | 1 | 35 | 0 | 0.5 | 1 | 60 | 1 | 0.75 | 1 |
| 11 | 1 | 0 | 0 | 36 | 0.5 | 0.5 | 0 | 61 | 0 | 1 | 0 |
| 12 | 1 | 0 | 0.25 | 37 | 0.5 | 0.5 | 0.25 | 62 | 0 | 1 | 0.25 |
| 13 | 1 | 0 | 0.5 | 38 | 0.5 | 0.5 | 0.5 | 63 | 0 | 1 | 0.5 |
| 14 | 1 | 0 | 0.75 | 39 | 0.5 | 0.5 | 0.75 | 64 | 0 | 1 | 0.75 |
| 15 | 1 | 0 | 1 | 40 | 0.5 | 0.5 | 1 | 65 | 0 | 1 | 1 |
| 16 | 0 | 0.25 | 0 | 41 | 1 | 0.5 | 0 | 66 | 0.5 | 1 | 0 |
| 17 | 0.5 | 0.25 | 0 | 42 | 1 | 0.5 | 0.25 | 67 | 0.5 | 1 | 0.25 |
| 18 | 1 | 0.25 | 0 | 43 | 1 | 0.5 | 0.5 | 68 | 0.5 | 1 | 0.5 |
| 19 | 0 | 0.25 | 0.25 | 44 | 1 | 0.5 | 0.75 | 69 | 0.5 | 1 | 0.75 |
| 20 | 0.5 | 0.25 | 0.25 | 45 | 1 | 0.5 | 1 | 70 | 0.5 | 1 | 1 |
| 21 | 1 | 0.25 | 0.25 | 46 | 0 | 0.75 | 0 | 71 | 1 | 1 | 0 |
| 22 | 0 | 0.25 | 0.5 | 47 | 0.5 | 0.75 | 0 | 72 | 1 | 1 | 0.25 |
| 23 | 0.5 | 0.25 | 0.5 | 48 | 1 | 0.75 | 0 | 73 | 1 | 1 | 0.5 |
| 24 | 1 | 0.25 | 0.5 | 49 | 0 | 0.75 | 0.25 | 74 | 1 | 1 | 0.75 |
| 25 | 0 | 0.25 | 0.75 | 50 | 0.5 | 0.75 | 0.25 | 75 | 1 | 1 | 1 |

EP 2 962 741 B1

## FIG. 12

| NUMBER | SHAFT WEIGHT | FLEX | KICK POINT |
|--------|--------------|------|------------|
| 1 | 0 | 0 | 0 |
| 2 | 0 | 0 | 0.25 |
| 3 | 0 | 0 | 0.5 |
| 4 | 0 | 0 | 0.75 |
| 5 | 0 | 0 | 1 |
| 6 | 0 | 0.25 | 0 |
| 7 | 0 | 0.25 | 0.25 |
| 8 | 0 | 0.25 | 0.5 |
| 9 | 0 | 0.25 | 0.75 |
| 10 | 0 | 0.25 | 1 |
| 11 | 0 | 0.5 | 0 |
| 12 | 0 | 0.5 | 0.25 |
| 13 | 0 | 0.5 | 0.5 |
| 14 | 0 | 0.5 | 0.75 |
| 15 | 0 | 0.5 | 1 |
| 16 | 0 | 0.75 | 0 |
| 17 | 0 | 0.75 | 0.25 |
| 18 | 0 | 0.75 | 0.5 |
| 19 | 0 | 0.75 | 0.75 |
| 20 | 0 | 0.75 | 1 |
| 21 | 0 | 1 | 0 |
| 22 | 0 | 1 | 0.25 |
| 23 | 0 | 1 | 0.5 |
| 24 | 0 | 1 | 0.75 |
| 25 | 0 | 1 | 1 |

| NUMBER | SHAFT WEIGHT | FLEX | KICK POINT |
|--------|--------------|------|------------|
| 26 | 0.5 | 0 | 0 |
| 27 | 0.5 | 0 | 0.25 |
| 28 | 0.5 | 0 | 0.5 |
| 29 | 0.5 | 0 | 0.75 |
| 30 | 0.5 | 0 | 1 |
| 31 | 0.5 | 0.25 | 0 |
| 32 | 0.5 | 0.25 | 0.25 |
| 33 | 0.5 | 0.25 | 0.5 |
| 34 | 0.5 | 0.25 | 0.75 |
| 35 | 0.5 | 0.25 | 1 |
| 36 | 0.5 | 0.5 | 0 |
| 37 | 0.5 | 0.5 | 0.25 |
| 38 | 0.5 | 0.5 | 0.5 |
| 39 | 0.5 | 0.5 | 0.75 |
| 40 | 0.5 | 0.5 | 1 |
| 41 | 0.5 | 0.75 | 0 |
| 42 | 0.5 | 0.75 | 0.25 |
| 43 | 0.5 | 0.75 | 0.5 |
| 44 | 0.5 | 0.75 | 0.75 |
| 45 | 0.5 | 0.75 | 1 |
| 46 | 0.5 | 1 | 0 |
| 47 | 0.5 | 1 | 0.25 |
| 48 | 0.5 | 1 | 0.5 |
| 49 | 0.5 | 1 | 0.75 |
| 50 | 0.5 | 1 | 1 |

| NUMBER | SHAFT WEIGHT | FLEX | KICK POINT |
|--------|--------------|------|------------|
| 51 | 1 | 0 | 0 |
| 52 | 1 | 0 | 0.25 |
| 53 | 1 | 0 | 0.5 |
| 54 | 1 | 0 | 0.75 |
| 55 | 1 | 0 | 1 |
| 56 | 1 | 0.25 | 0 |
| 57 | 1 | 0.25 | 0.25 |
| 58 | 1 | 0.25 | 0.5 |
| 59 | 1 | 0.25 | 0.75 |
| 60 | 1 | 0.25 | 1 |
| 61 | 1 | 0.5 | 0 |
| 62 | 1 | 0.5 | 0.25 |
| 63 | 1 | 0.5 | 0.5 |
| 64 | 1 | 0.5 | 0.75 |
| 65 | 1 | 0.5 | 1 |
| 66 | 1 | 0.75 | 0 |
| 67 | 1 | 0.75 | 0.25 |
| 68 | 1 | 0.75 | 0.5 |
| 69 | 1 | 0.75 | 0.75 |
| 70 | 1 | 0.75 | 1 |
| 71 | 1 | 1 | 0 |
| 72 | 1 | 1 | 0.25 |
| 73 | 1 | 1 | 0.5 |
| 74 | 1 | 1 | 0.75 |
| 75 | 1 | 1 | 1 |

EP 2 962 741 B1

# FIG. 13

EP 2 962 741 B1

FIG. 14

EP 2 962 741 B1

# FIG. 15

| High Fade | High Straight | High Draw |
|-----------|---------------|-----------|
| Mid Fade | Mid Straight | Mid Draw |
| Low Fade | Low Straight | Low Draw |

# FIG. 16

Head Speed:Max
V. Trajectory:Low
H. Trajectory:Draw

| High Fade | High Straight | High Draw |
|-----------|---------------|-----------|
| Mid Fade | Mid Straight | Mid Draw |
| Low Fade | Low Straight | Low Draw |

Low-Draw DNA

Weight = 1.0  Flex= 0.25  Pitch=0.5

# FIG. 17

| | DESIGN VARIABLES | DEGREE OF IMPACT |
|---|---|---|
| SHAFT | FLEXURAL RIGIDITY | 4 |
| | TORSIONAL RIGIDITY | 3 |
| | WEIGHT | 3 |
| | FLEXURAL RIGIDITY DISTRIBUTION | 3 |
| | TORSIONAL RIGIDITY DISTRIBUTION | 1 |
| | WEIGHT DISTRIBUTION | 3 |
| CLUB | TOTAL WEIGHT | 5 |
| | BALANCE | 3 |
| | STIFFNESS | 4 |
| | LENGTH | 5 |
| HEAD | WEIGHT | 5 |
| | DEPTH OF GRAVITY CENTER | 3 |
| | HEIGHT OF GRAVITY CENTER | 1 |
| | DISTANCE OF GRAVITY CENTER | 3 |
| | ANGLE OF GRAVITY CENTER | 3 |
| | LOFT ANGLE | 2 |
| | LIE ANGLE | 2 |
| | FACE ANGLE | 2 |
| GRIP | WEIGHT | 3 |
| | DIAMETER | 3 |
| | STIFFNESS | 3 |

FIG. 18

CLUB SPEED [m/s]

HEIGHT OF GRAVITY CENTER OF HEAD

TORSIONAL RIGIDITY DISTRIBUTION OF SHAFT

LOW MEDIUM HIGH

HIGH

MEDIUM

195g

200g

HEAD WEIGHT

205g

PLOTTED NUMBER

**EP 2 962 741 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013037493 A
- JP 2013082102 A
- JP 2005312734 A
- JP 4871218 B
- JP 2011000425 A